(19) Europäisches Patentamt
European Patent Office
Office européen des brevets

(11) Numéro de publication: 0 083 894 B1

(12) **FASCICULE DE BREVET EUROPEEN**

(45) Date de publication du fascicule du brevet: **20.03.85**

(51) Int. Cl.⁴: **C 07 C 179/10**, C 07 C 178/00

(21) Numéro de dépôt: **82402395.6**

(22) Date de dépôt: **29.12.82**

(54) **Perfectionnement aux procédés de synthèse des acides percarboxyliques.**

(30) Priorité: **13.01.82 FR 8200407**

(43) Date de publication de la demande: **20.07.83 Bulletin 83/29**

(45) Mention de la délivrance du brevet: **20.03.85 Bulletin 85/12**

(84) Etats contractants désignés: **BE CH DE FR GB IT LI LU NL SE**

(56) Documents cités:
**EP - A - 0 004 407**
**FR - A - 1 183 067**
**FR - A - 1 390 068**

(73) Titulaire: **ATOCHEM, 12/16, allée des Vosges, F-92400 Courbevoie (FR)**

(72) Inventeur: **Pralus, Michèle, 3, rue des Gosses, F-69450 Saint-Cyr au Mont d'Or (FR)**
Inventeur: **Lecoq, Jean-Claude, 10 A, rue Josserand, *F-69630 Chaponost (FR)***
Inventeur: **Schirmann, Jean-Pierre, 49, Chemin de la Glacière, F-69600 Oullins (FR)**

(74) Mandataire: **Rochet, Michel et al, ATOCHEM Département Propriété Industrielle Cédex 22, F-92091 Paris la Défense (FR)**

**EP 0 083 894 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un perfectionnement aux procédés de préparation de solutions organiques anhydres d'acides percarboxyliques par réaction de solutions aqueuses de peroxyde d'hydrogène avec des acides carboxyliques miscibles à l'eau, où l'on opère en présence d'un catalyseur et où l'eau introduite avec le peroxyde d'hydrogène et l'eau formée au cours de la réaction est éliminée en continu par distillation azéotropique au moyen d'un solvant organique susceptible de former un hétéroazéotrope avec l'eau.

La réaction du peroxyde d'hydrogène avec les acides carboxyliques est une réaction équilibrée, qui s'écrit:

$$R-\underset{\underset{O}{\|}}{C}-OH + H_2O_2 \rightleftharpoons R-\underset{\underset{O}{\|}}{C}-O-OH + H_2O \quad (1)$$

Cette réaction doit en général être conduite à température modérée en raison de l'instabilité des acides percarboxyliques formés. Pour l'accélérer, on a recours à des catalyseurs, qui sont le plus souvent des acides minéraux ou organiques forts, comme l'acide phosphorique, l'acide sulfurique, l'acide chlorhydrique, les acides alkyl- ou arylsulfoniques, l'acide trifluoracétique ou les résines cationiques acides.

Pour déplacer vers la droite l'équilibre de la réaction 1, il a été proposé, par exemple dans les brevets des Etats-Unis d'Amérique N$^{os}$ 2877266 et 2814641, d'éliminer l'eau en continu par distillation azéotropique. De son côté, la titulaire a montré que, dans ce cas, l'on pouvait avantageusement remplacer le catalyseur acide fort par un oxyde métalloïdique (demande de brevet européen N° 22396) ou par un acide borique (demande de brevet européen N° 25381).

Ces techniques de préparation des acides percarboxyliques utilisant la distillation hétéroazéotropique de l'eau au cours de la réaction présentent néanmoins un inconvénient important: l'entraînement dans la phase aqueuse distillée d'une quantité non négligeable d'oxygène peroxydique. L'oxygène peroxydique entraîné est sous forme de peroxyde d'hydrogène non transformé et/ou d'acide percarboxylique. Ce phénomène d'entraînement d'oxygène peroxydique dans la phase aqueuse distillée se manifeste quel que soit le catalyseur utilisé. Les pertes par entraînement peuvent atteindre jusqu'à 4% de la quantité de peroxyde d'hydrogène engagée, comme l'ont montré B. Philips *et al.*, dans «Journal of Organic Chemistry», *23,* 1923 (1958), et M. Hrusovsky, dans «Chemical Abstracts», *78,* 123937.

A ces pertes par entraînement dans la phase aqueuse distillée s'ajoutent des pertes appréciables de peroxyde d'hydrogène par décomposition dans la colonne de distillation azéotropique. Dans cette colonne, le peroxyde d'hydrogène entraîné avec l'hétéroazéotrope eau/solvant organique est en effet soumis à des conditions très propices à sa décomposition, d'autant plus qu'il est alors soustrait à l'action protectrice d'un éventuel stabilisant introduit initialement dans le mélange réactionnel.

Les pertes en oxygène peroxydique dues à l'entraînement dans la phase aqueuse distillée et aux décompositions dans la colonne de distillation azéotropique grèvent l'économie de ces procédés de préparation des acides percarboxyliques.

Dans toutes les techniques antérieures, le réacteur où s'effectue la réaction du peroxyde d'hydrogène avec l'acide carboxylique, en présence du catalyseur et du solvant organique servant d'entraîneur azéotropique, est surmonté d'une colonne de distillation, munie d'un condenseur et d'un décanteur. Seule la phase organique est refluée dans la colonne, tandis que la phase aqueuse décantée est soutirée en continu.

La titulaire vient de découvrir qu'il est possible de réduire considérablement les pertes en oxygène peroxydique dans la colonne de distillation azéotropique en injectant en tête de cette colonne une quantité d'eau telle que le reflux liquide ait une composition voisine de celle des vapeurs de l'hétéroazéotrope eau/solvant organique. Lorsqu'on opère en discontinu, cette injection d'eau est poursuivie pendant la plus grande partie du temps de réaction. Elle est ensuite arrêtée et la distillation azéotrope est continuée en refluant seulement la phase organique provenant de la condensation des vapeurs de l'hétéroazéotrope jusqu'à ce que la solution d'acide percarboxylique dans le réacteur soit pratiquement anhydre.

Le débit d'eau injectée en tête de la colonne de distillation azéotrope est fonction de la composition de l'hétéroazéotrope et dépend donc essentiellement de la nature du solvant organique servant d'entraîneur azéotropique et, dans une moindre mesure, de la pression de fonctionnement de la colonne de distillation. Ce débit d'eau injectée doit toujours être inférieur à la quantité d'eau soutirée au condenseur. Il est de préférence égal à la moitié du débit de la phase aqueuse soutirée. On peut ainsi opérer facilement en continu et obtenir une solution organique d'acide percarboxylique contenant moins de 0,3% d'eau en poids. L'eau injectée peut être de l'eau pure; ce peut être aussi une partie de la phase aqueuse provenant de la décantation de l'hétéroazéotrope condensé.

L'intérêt d'une réinjection d'eau dans une colonne destinée à déshydrater le milieu réactionnel n'était pas du tout évident, car l'on pouvait redouter un allongement considérable du temps de réaction. Il n'en est rien et la titulaire a même constaté qu'en appliquant le perfectionnement objet de l'invention à une réaction catalysée par l'acide sulfurique, il est possible de réduire la quantité de ce catalyseur à des valeurs très inférieures à celles habituellement utilisées, en conservant sensiblement la même vitesse de réaction. C'est ainsi que l'on peut travailler avec des proportions d'acide sulfurique allant de 0,0005 à 0,010 mol par mole de peroxyde d'hydrogène au lieu des proportions au moins 10 fois supérieures préconisées par l'art antérieur.

L'importante réduction des pertes en oxygène peroxydique obtenue selon l'invention conduit à une meilleure utilisation du peroxyde d'hydrogène, qui se trouve refoulé en pied de colonne et peut ainsi être transformé à peu près quantitativement en acide percarboxylique.

Les exemples suivants, donnés à titre non limitatif, illustrent l'application du procédé selon l'invention dans des réactions de préparation de l'acide perpropionique catalysées par l'acide sulfurique ou l'acide borique. Des résultats analogues sont obtenus avec les acides acétique ou butyrique, ou avec d'autres catalyseurs, comme les résines cationiques acides fortes ou les oxydes métalloïdiques.

*Exemple 1:*

Dans un réacteur en verre de 1 l, équipé d'une colonne de distillation de 15 plateaux Oldershaw et d'un condenseur à reflux avec système de décantation, on introduit 630 g d'une solution ayant la composition suivante:

| | |
|---|---|
| dichloro-1,2-éthane | 20% en poids |
| acide propionique | 79,95% en poids |
| acide sulfurique | 0,05% en poids |

Ce mélange est porté à l'ébullition à reflux sous une pression de 100 mm de mercure (13,3 kPa). On introduit alors dans le réacteur en 20 min 92 g d'une solution aqueuse à 69,6% en poids de peroxyde d'hydrogène, contenant également 0,8% en poids d'acide dipicolinique servant de stabilisant. Simultanément on injecte en tête de la colonne de distillation, pendant 2 h, 25 g/h d'eau. La température du réacteur est de 66° C. La phase organique condensée est recyclée en tête de colonne pour assurer le reflux. La phase aqueuse condensée est séparée par décantation et soutirée en continu.

On arrête la réaction au bout de 2½ h. La phase aqueuse distillée, qui pèse 108 g, contient 0,05% en poids de peroxyde d'hydrogène, ce qui représente 0,084% de la quantité de peroxyde d'hydrogène engagé dans la réaction.

La solution organique d'acide percarboxylique obtenue contient 24,9% en poids d'acide perpropionique, 0,2% en poids de peroxyde d'hydrogène et 0,11% en poids d'eau.

Le taux de transformation du peroxyde d'hydrogène en acide perpropionique atteint 95,9%. Les pertes totales en peroxyde d'hydrogène ne sont que de 1,8%.

*Exemple 2:*

Dans le réacteur utilisé à l'exemple 1, on place 636 g d'une solution contenant:

| | |
|---|---|
| dichloro-1,2-éthane | 19,8% en poids |
| acide propionique | 79,2% en poids |
| acide orthoborique | 1,0% en poids |

La solution est portée à l'ébullition à reflux sous une pression de 100 mm de mercure (13,3 kPa). On introduit alors dans le réacteur en 20 min 92 g d'une solution aqueuse à 70% en poids de peroxyde d'hydrogène, contenant également 0,7% en poids d'acide dipicolinique. En tête de la colonne de distillation on injecte en continu 21 g/h d'eau pendant 2½ h. La température du réacteur est de 65° C. Les vapeurs de l'hétéroazéotrope du dichloro-1,2-éthane et de l'eau sont condensées et décantées. Le dichloro-1,2-éthane est reflué en tête de la colonne de distillation, tandis que la phase aqueuse est soutirée en continu.

On arrête la réaction au bout de 3 h. La solution organique de peracide obtenue contient 23,7% en poids d'acide perpropionique, ce qui représente un taux de transformation de 92% du peroxyde d'hydrogène engagé. Les pertes en peroxyde d'hydrogène par entraînement dans la distillation sont de 0,12% et les pertes par décomposition de 5,4%.

*Exemple 3:*

Dans le réacteur utilisé à l'exemple 1, on introduit 636 g d'une solution ayant la composition suivante:

| | |
|---|---|
| dichloro-1,2-éthane | 50,7% en poids |
| acide propionique | 48,3% en poids |
| acide orthoborique | 1,0% en poids |

La solution est portée à l'ébullition à reflux sous une pression de 250 mm de mercure (33,3 kPa). On coule dans le réacteur en 15 min 60 g d'une solution aqueuse contenant 69,5% en poids de peroxyde d'hydrogène et 0,8% en poids d'acide dipicolinique. On injecte également pendant 2 h en tête de colonne un total de 42 g d'eau. La température du réacteur est de 66° C.

La réaction est arrêtée au bout de 3 h. La phase aqueuse séparée après condensation des vapeurs de l'hétéroazéotrope pèse 86 g et contient 1% en poids de peroxyde d'hydrogène, soit environ 2% de la quantité engagée initialement.

La solution organique de peracide obtenue contient 15,4% en poids d'acide perpropionique et 0,2% en poids de peroxyde d'hydrogène.

Le rendement en peracide par rapport au peroxyde d'hydrogène est de 90,5%. Les pertes totales en peroxyde d'hydrogène par décomposition et par entraînement sont de 6%.

*Exemple 4* (exemple comparatif):

On reproduit exactement l'exemple 3, mais en omettant l'injection d'eau en tête de colonne de distillation. On obtient dans ces conditions un rendement de 84,9% en acide perpropionique par rapport au peroxyde d'hydrogène. Les pertes totales en peroxyde d'hydrogène par décomposition et par entraînement atteignent 10,7%.

**Revendications**

1. Procédé perfectionné de préparation de solutions organiques pratiquement anhydres d'acides percarboxyliques par réaction de solutions aqueuses de peroxyde d'hydrogène avec des acides carboxyliques miscibles à l'eau, en présence d'un catalyseur et d'un solvant organique permettant l'élimination continue par distillation

azéotropique de l'eau du milieu réactionnel, caractérisé en ce que l'on injecte en continu en tête de la colonne de distillation azéotropique, pendant la plus grande partie de la réaction, une quantité d'eau telle que le reflux liquide ait une composition voisine de celle des vapeurs de l'hétéroazéotrope eau/solvant organique.

2. Procédé selon la revendication 1, caractérisé en ce que l'eau injectée est de l'eau pure.

3. Procédé selon la revendication 1, caractérisé en ce que l'eau injectée provient de la phase aqueuse séparée après condensation des vapeurs de l'hétéroazéotrope.

5. Procédé selon l'une des revendications 1 à 3, caractérisé en ce que le débit de l'eau injectée est égal à environ la moitié du débit de l'eau séparée après condensation des vapeurs de l'hétéroazéotrope.

5. Procédé selon l'une des revendications 1 à 4, caractérisé en ce que le catalyseur de la réaction de formation de l'acide percarboxylique est un acide fort.

6. Procédé selon la revendication 5, caractérisé en ce que le catalyseur acide fort est l'acide sulfurique, utilisé à raison de 0,0005 à 0,010 mol par mole de peroxyde d'hydrogène.

7. Procédé selon l'une des revendications 1 à 4, caractérisé en ce que le catalyseur de formation de l'acide percarboxylique est un oxyde métalloïdique.

8. Procédé selon l'une des revendications 1 à 4, caractérisé en ce que le catalyseur de formation de l'acide percarboxylique est un acide borique.

## Patentansprüche

1. Verbessertes Verfahren zur Herstellung von praktisch wasserfreien organischen Lösungen von Percarbonsäure durch Umsetzung von wässerigen Wasserstoffperoxidlösungen mit wassermischbaren Percarbonsäuren in Gegenwart eines Katalysators und eines organischen Lösungsmittels, das die kontinuierlich Entfernung des Wassers aus dem Reaktionsmilieu durch azeotrope Destillation ermöglicht, dadurch gekennzeichnet, dass man während des grössten Teils der Umsetzung kontinuierlich am Kopf der Kolonne zur azeotropen Destillation eine derartige Menge Wasser zuführt, dass der flüssige Rückfluss eine Zusammensetzung aufweist, die derjenigen der Dämpfe des Heteroazeotrops Wasser/organisches Lösungsmittel ähnlich ist.

2. Verfahren nach Anspruch 1, dadurch gekennzeichnet, dass das zugeführte Wasser reines Wasser ist.

3. Verfahren nach Anspruch 1, dadurch gekennzeichnet, dass das zugeführte Wasser aus der wässerigen Phase stammt, die nach Kondensation der Dämpfe des Heteroazeotrops abgetrennt wird.

4. Verfahren nach einem der Ansprüche 1 bis 3, dadurch gekennzeichnet, dass die Menge des zugeführten Wassers etwa der Hälfte der Menge des Wassers entspricht, das nach Kondensation der Dämpfe des Heteroazeotrops abgetrennt wird.

5. Verfahren nach einem der Ansprüche 1 bis 4, dadurch gekennzeichnet, dass der Katalysator der Bildungsreaktion der Percabonsäure eine starke Säure ist.

6. Verfahren nach Anspruch 5, dadurch gekennzeichnet, dass die katalysierende starke Säure Schwefelsäure ist, die in Mengen zwischen 0,0005 und 0,010 mol pro Mol Wasserstoffperoxid eingesetzt wird.

7. Verfahren nach einem der Ansprüche 1 bis 4, dadurch gekennzeichnet, dass der Katalysator der Bildung von Percarbonsäure ein Oxid eines Halbmetalls ist.

8. Verfahren nach einem der Ansprüche 1 bis 4, dadurch gekennzeichnet, dass der Katalysator der Bildung der Percarbonsäure eine Borsäure ist.

## Claims

1. Improved process for the preparation of organic, virtually anhydrous, solutions of percarboxylic acids by reaction of aqueous hydrogen peroxide solutions with water-miscible carboxylic acids in the presence of a catalyst and an organic solvent, allowing continuous removal of the water from the reaction mixture by azeotropic distillation, characterised in that at the top of the azeotropic distillation column there is injected continuously, over the course of the greater part of the reaction, an amount of water which is such that the liquid reflux has a composition close to that of the vapours of the water/organic solvent hetero-azeotrope.

2. Process according to Claim 1, characterised in that the water injected is pure water.

3. Process according to Claim 1, characterised in that the injected water originates from the aqueous phase which has been separated off after condensing the vapours of the hetero-azeotrope.

4. Process according to any one of Claims 1 to 3, characterised in that the rate of injection of water is equal to about half the rate of separating-off of water after condensation of the vapours of the hetero-azeotrope.

5. Process according to any one of Claims 1 to 4, characterised in that the catalyst for the percarboxylic acid formation reaction is a strong acid.

6. Process according to Claim 5, characterised in that the strong acid catalyst is sulphuric acid used in an amount of 0.0005 to 0.010 mol per mole of hydrogen peroxide.

7. Process according to any one of Claims 1 to 4, characterised in that the percarboxylic acid formation catalyst is a metalloid oxide.

8. Process according to any one of Claims 1 to 4, characterised in that the percarboxylic acid formation catalyst is a boric acid.